# EUROPEAN PATENT SPECIFICATION

(11) **EP 3 563 382 B1**
(45) Date of publication and mention of the grant of the patent: **12.06.2024**
(21) Application number: 18700973.3
(22) Date of filing: 02.01.2018
(51) Int. Cl.: G16H 40/67

(54) **CONNECTION UNIT FOR CONNECTING A PLURALITY OF MEDICAL DEVICES AND SYSTEM COMPRISING SAME**
VERBINDUNGSEINHEIT ZUM VERBINDEN MEHRERER MEDIZINISCHER VORRICHTUNGEN UND SYSTEM WELCHES SELBIGE BEINHALTET
UNITÉ DE CONNEXION POUR UNE PLURALITÉ DE DISPOSITIFS MÉDICAUX ET SYSTÈME LA COMPRENANT

(30) Priority: 30.12.2016 EP 16207633; 22.12.2017 EP 17210554
(43) Date of publication of application: 06.11.2019
(73) Proprietor: Televic Healthcare NV, 8870 Izegem (BE)
(72) Inventor: CROMBEZ, Pieter, B-8820 Torhout (BE); VERHAEGHE, Geert, B-8680 Koekelare (BE); VERHEYE, Marino, B-8870 Izegem (BE)
(74) Representative: Beck, Michaël Andries T.
(86) International application number: PCT/EP2018/050081
(87) International publication number: WO 2018/122416

(56) References cited:
- US-A1- 2010 234 718
- US-A1- 2012 025 991
- US-A1- 2013 045 685

## Description

### Field of the Invention

The present invention pertains to the field of patient monitoring equipment, and in particular to the field of connection units for patient monitoring devices.

### Background

In hospitals, patients may be monitored by one or more medical devices that may be configured to sound an alarm when a certain vital parameter, observed by means of a sensor of the medical device, crosses a predetermined threshold, or when certain anomalies occur in the monitoring equipment (such as a cable being disconnected, or network connection being lost). The resulting alarm signals must be relayed to the relevant medical personnel, to ensure that adequate action is taken in a timely manner. Today, the relaying of such signals relies on dedicated networks, all components of which must be certified as medical devices according to applicable standards and regulations.

US 2010/234718 discloses sensors connected via a network to a sensor processing module configured to generate alarms in response to physiological parameters exceeding certain safe thresholds and relaying alarms to electronic devices of emergency personnel, wherein said relaying can be activated/deactivated by authorized personnel. It does not disclose a delayed activation command allowing a respective one of said plurality of medical devices to be connected to a medical interface without relaying any alarms for a predetermined time interval, nor an authentication unit provided with means to authenticate authorized personnel, prior to transmitting said control signals to said electronic circuitry.

US2012025991 relates to bed exit alarms.

US2013045685 relates to patient monitors providing alarms to multi-patient monitoring systems.

### Summary of the invention

According to an aspect of the present invention, there is provided a connection unit for connecting a plurality of medical devices to a nurse call network, the connection unit comprising: a plurality of medical device interfaces, adapted to receive respective cables connecting to respective ones of said plurality of medical and detected losses of connectivity to said nurse call network over said network interface; and selectively activate and deactivate said monitoring and said relaying upon receipt of predetermined control signals over said control interface.

The connection unit further comprises an authentication unit interface configured for receiving authentication unit signals from an authentication unit and providing said authentication unit signals as control signals to said electronic circuitry. The authentication unit signals and the control signals comprise medical device port selection information. The medical device port selection information comprises, for one or more of said plurality of medical device ports, a delayed activation command.

It is an advantage of the invention that critical operations such as activating and deactivating a medical device interface can be reserved to specific authorized persons, who must be authenticated prior to be allowed to trigger control signals. It is a further advantage that by delaying the activation, certain additional procedures can be performed - e.g. local testing of the attached device -before the port becomes fully active.

The electronic circuitry may be implemented as a single non-programmable electronic device. The term "single non-programmable electronic device" is used herein to describe an electronic component that does not provide the aforementioned functions by storing a user-definable list of instructions (i.e., a program) and executing these instructions, but relies on the operation of a fixed network of logic gates. Thus, the term "single non-programmable electronic device" includes for example ASICs and FPGAs that are not configured to act upon lists of instructions, and excludes microcontrollers and microprocessors that require software to perform any meaningful task.

It is an advantage thereof that reliable networked monitoring of medical devices can be provided over pre-existing nurse call networking infrastructure without a need for certification of new software.

Alternatively, the electronic circuitry may be implemented as a programmable electronic device, such as a general-purpose processor, a microcontroller, or a DSP, programmed with adequate instructions, or as a combination of several programmable and/or non-programmable devices.

In an embodiment, the connection unit according to the present invention further comprises an indicator adapted to signal an alarm condition, said electronic circuitry is further operatively connected to said indicator, and said electronic circuitry is further configured to control said indicator to signal said alarm condition upon said receipt of device alarm signals and losses of connectivity. In a particular embodiment, the indicator is adapted to signal said alarm by visual and/or auditory means.

It is an advantage of this embodiment that alarms can be notified to the locally present personnel, independently of and in addition to the relaying of the alarms over the network.

In an embodiment of the connection unit according to the present invention, said electronic circuitry is further configured to monitor a loss of connectivity at said network interface, and to activate a local alarm status when said loss of connectivity at said network interface is detected. In a particular embodiment, the connection unit further comprises a room lamp interface, and said electronic circuitry is further configured to send an alarm light signal to said room lamp interface when said local alarm status is activated.

It is an advantage of this embodiment that the cluster of monitored medical devices can be operated in a "local mode" when the network is not available. In addition, by triggering a visual alarm on the room lamp, personnel outside the patient room is immediately made aware of the loss of network connectivity and can take appropriate corrective action.

The connection unit according to the present invention further comprises an authentication unit interface configured for receiving authentication unit signals from an authentication unit and providing said authentication unit signals as control signals to said electronic circuitry. In a particular embodiment, said authentication unit signals and said control signals comprise medical device port selection information. In a particular embodiment, said medical device port selection information comprises, for one or more of said plurality of medical device ports, one of a group of an activation command and a deactivation command.

It is an advantage of this embodiment that critical operations such as activating and deactivating a medical device interface can be reserved to specific authorized persons, who must be authenticated prior to be allowed to trigger control signals.

According to an aspect of the present invention, there is provided a system comprising a connection unit as described above and at least one medical device connected to one of said medical device interfaces.

In an embodiment, the system according to the present invention further comprises an authentication unit operatively connected to said authentication unit interface.

### Brief Description of the Figures

These and other technical features and advantages of embodiments of the present invention will now be described with reference to the accompanying figure, which schematically illustrates a connection unit according to an embodiment of the present invention.

### Description of Embodiments

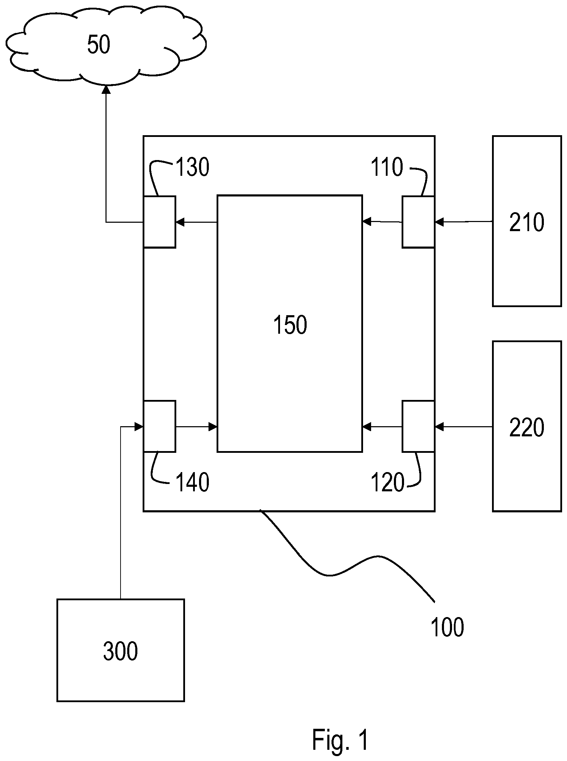

Figure 1 schematically illustrates a connection unit **100** for connecting a plurality of medical devices to a nurse call network, according to an embodiment of the present invention.

The connection unit **100** comprises a plurality of medical device interfaces to receive cables connecting to different medical devices. Without loss of generality, only two medical device interfaces **110, 120** are shown in Figure 1, having two respective medical devices **210, 220** connected to them, by means of appropriate cables and connectors (not shown in detail). The connection unit **100** may be designed in a modular way, whereby each module has a fixed number of medical device interfaces (e.g., two), and multiple modules may be interconnected to obtain a combined connection unit **100** having a larger total number of medical device interfaces. The connection unit **100** is preferably provided with status indicators (such as color-coded LEDs) to visually indicate the unit's status, which may include "on" (active), "off" (inactive), "stand-by", "error/fault", and the like (not shown in the schematic illustration). The individual medical device interfaces or ports may also be provided with status indicators (such as color-coded LEDs) to visually indicate the port's status, which may include "inactive", "active - no connection", "active - connected", "active - alarm", and the like (not shown in the schematic illustration).

The medical device interfaces **110, 120** allow the connection unit **100** to receive data (in particular medical sensor data or information derived from medical sensor data) from the attached medical devices **210, 220,** and may further allow the connection unit **100** to transmit data (in particular control data and commands affecting the operation of the devices) to the attached medical devices **210, 220.**

The connection unit **100** further comprises a network interface **130,** adapted to connect to a nurse call network **50.** A nurse call network is a network suitable for connecting patient room terminals to a central monitoring station, thus allowing patients to call for assistance when necessary. It is an advantage of the present invention that the same network, which may already be present in the premises, can be used for relaying signals from medical devices **210, 220** to the monitoring station.

The connection unit **100** further comprises a control interface, as will be described in more detail below.

The connection unit **100** further comprises electronic circuitry **150,** operatively connected to said plurality of medical device interfaces **110, 120,** said network interface **130,** and said control interface. Without loss of generality, the illustrated electronic circuitry **150** is implemented as a single non-programmable electronic device.

The electronic circuitry **150** is configured to monitor receipt of device alarm signals and losses of connectivity at the medical device interfaces **110, 120.** In order to allow detection of losses of connectivity, the medical devices **210, 220** must either send a permanent or intermittent keep-alive or "heartbeat" signal, or provide an electronic loopback of a test signal emitted by the medical device interfaces **110, 120.**

The electronic circuitry **150** is further configured to relay any received device alarm signals and any detected losses of connectivity to the nurse call network **50** over the network interface **130,** such that appropriate action can be taken by the staff that monitors the incoming alarms. The monitoring and the relaying are selectively activated and deactivated upon receipt of predetermined control signals over the control interface **140.** In this way, individual device interfaces **110, 120** are turned on or off by means of appropriate control signals - this is useful to deactivate a port (device interface) that is not connected to an active device, and that might otherwise generate a permanent "loss of signal" alarm.

The connection unit **100** may further comprise an indicator adapted to signal an alarm condition, for example by visual and/or auditory means. This is useful when the alarm must be notified to staff present in the same room with the connection unit **100,** in addition to or alternatively to its being relayed over the nurse call network **50.** To this end, the electronic circuitry **150** is further operatively connected to the indicator, and the electronic circuitry **150** is further configured to control the indicator to signal the alarm condition upon receipt of device alarm signals and losses of connectivity.

The electronic circuitry **150** may be further configured to monitor a loss of connectivity at the network interface **130,** and to activate a local alarm status when loss of connectivity at the network interface **130** is detected. The local alarm status, which may be signaled by visual and/or auditory effects, provides a way to attract attention to the fact that the network connection is lost (which implies that relaying alarm signals to a centralized monitoring station is not possible). Preferably, the connection unit **100** also comprises a room lamp interface, and the connection unit **100** is further configured to send an alarm light signal to the room lamp interface when the local alarm status is activated. A room lamp or overdoor lamp is typically provided outside a patient room (e.g., above the door opening, in the hallway or corridor) to visually signal the presence of a call or an alarm situation to staff present outside the patient rooms. A room lamp may be capable of signaling various different states, by using different colors of light (or combinations of colors), flashing patterns, etc. The room lamp interface is preferably adapted to provide the necessary control signals to the room lamp to operate in accordance with various different states.

The connection unit **100** further comprises an authentication unit interface configured for receiving authentication unit signals from an authentication unit **300** and providing these authentication unit signals as control signals to the electronic circuitry **150.** Thus, the authentication unit interface is an external interface which connects to an internal control interface **140.** The authentication unit interface also comprises the control interface **140,** in the sense that it may be configured to relay both control signals and other types of signals. The authentication unit signals and the control signals may include any signals that control the operation of the connection unit **100,** such as signals to activate or deactivate the connection unit **100** as a whole. The authentication unit signals and the control signals also comprise medical device port selection information, such as, for one or more of said plurality of medical device ports, one of a group of an activation command, a delayed activation command, and a deactivation command. The deactivation command allows one of the medical device interface to be turned off, specifically when no active medical device is connected to it, while the rest of the connection unit **100** remains operational. The delayed activation command allows a medical device to be connected to a medical device interface without relaying any alarms for a predetermined time interval - the time interval may be in the order of one minute and may be used to locally generate a test alarm on the connected medical device without causing the alarm to be relayed and emergency personnel to be called in.

The authentication unit **100** is provided with means to authenticate authorized personnel, prior to transmitting control signals to the electronic circuitry **150.** In the context of medical device port selection information, this avoids the risk of having patients or other non-authorized persons deactivate the monitoring of certain medical devices that gather critical patient data. The means to authenticate may include a keypad for receiving a PIN or a password, a biometric sensor such as a fingerprint scanner, an iris scanner, or a voice recognition device, a chip card reader, an RFID reader, a magnetic induction transceiver, and the like. The connection unit **100** may be part of a system, along with at least one medical device **210, 220** connected to one of its medical device interfaces **110, 120,** and optionally with an authentication unit **300** operatively connected to the authentication unit interface.

While the invention has been described hereinabove with reference to specific embodiments, this was done to illustrate and not to limit the invention, the scope of which is to be determined by reference to the attached claims.

## Claims

1. A connection unit (100) for connecting a plurality of medical devices to a nurse call network, the connection unit comprising:
- a plurality of medical device interfaces (110, 120), adapted to receive respective cables connecting to respective ones of said plurality of medical devices (210, 220);
- a network interface (130), adapted to connect to said nurse call network;
- an internal control interface (140);
- electronic circuitry (150), operatively connected to said plurality of medical device interfaces (110, 120), said network interface (130), and said internal control interface (140), said electronic circuitry (150) being configured to:
o monitor receipt of device alarm signals and losses of connectivity at said plurality of medical device interfaces (110, 120);
o relay received device alarm signals and detected losses of connectivity to said nurse call network over said network interface (130); and
o selectively activate and deactivate said monitoring and said relaying upon receipt of predetermined control signals over said internal control interface; and
- an external authentication unit interface configured for receiving authentication unit signals from an authentication unit (300) and providing said authentication unit signals as control signals to said electronic circuitry (150) over said internal control interface (140), whereby said authentication unit (300) is provided with means to authenticate authorized personnel, prior to transmitting said control signals to said electronic circuitry (150);
wherein said authentication unit signals and said control signals comprise medical device interface selection information; and wherein said medical device interface selection information comprises, for one or more of said plurality of medical device interfaces (110, 120), a delayed activation command;
said delayed activation command allowing a respective one of said plurality of medical devices (210, 220) to be connected to a medical device interface (110, 120) without relaying any alarms for a predetermined time interval.

2. The connection unit (100) according to claim 1, wherein said electronic circuitry (150) is implemented as a single non-programmable electronic device.

3. The connection unit (100) according to claim 1 or claim 2, further comprising an indicator adapted to signal an alarm condition, wherein said electronic circuitry (150) is further operatively connected to said indicator, and wherein said electronic circuitry (150) is further configured to control said indicator to signal said alarm condition upon said receipt of device alarm signals and losses of connectivity.

4. The connection unit (100) according to claim 3, wherein said indicator is adapted to signal said alarm by visual and/or auditory means.

5. The connection unit (100) according to any of the preceding claims, wherein said electronic circuitry (150) is further configured to monitor a loss of connectivity at said network interface (130), and to activate a local alarm status when said loss of connectivity at said network interface (130) is detected.

6. The connection unit (100) according to claim 5, further comprising a room lamp interface, wherein said electronic circuitry (150) is further configured to send an alarm light signal to said room lamp interface when said local alarm status is activated.

7. The connection unit (100) according to any of the preceding claims, wherein said medical device interface selection information comprises, for one or more of said plurality of medical device interfaces, one of a group of an activation command and a deactivation command.

8. A system comprising a connection unit (100) according to any of the preceding claims and at least one medical device (210, 220) connected to one of said medical device interfaces (110, 120).

9. The system according to claim 8, further comprising an authentication unit (300) operatively connected to said authentication unit interface.

## Patentansprüche

1. Verbindungseinheit (100) zum Verbinden mehrerer medizinischer Geräte mit einem Krankenpflegepersonalrufnetz, wobei die Verbindungseinheit Folgendes aufweist:
- mehrere medizinische Geräte-Schnittstellen (110, 120), die ausgebildet sind, jeweilige Kabel zur Verbindung mit den jeweiligen der mehreren medizinischen Geräte (210, 220) anzuschließen;
- eine Netz-Schnittstelle (130), die ausgebildet ist, mit dem Krankenpflegepersonalrufnetz zu verbinden;
- eine interne Steuerungs-Schnittstelle (140);
- eine elektronische Schaltung (150), die betriebsmäßig mit den mehreren medizinischen Geräte-Schnittstellen (110, 120), der Netz-Schnittstelle (130) und der internen Steuerungs-Schnittstelle (140) verbunden ist, wobei die elektronische Schaltung (150) ausgebildet ist:
∘ den Empfang von Gerätewarnsignalen und Verlusten der Konnektivität an den mehreren medizinischen Geräte-Schnittstellen (110, 120) zu überwachen;
∘ empfangene Gerätewarnsignale und erfasste Verluste der Konnektivität über die Netz-Schnittstelle (130) an das Krankenpflegepersonalrufnetz zu übertragen; und
∘ selektiv die Überwachung und die Übertragung bei Empfang von vorbestimmten Steuersignalen über die interne Steuerungs-Schnittstelle zu aktivieren und deaktivieren; und
- eine externe Authentifizierungseinheits-Schnittstelle, die zum Empfangen von Authentifizierungseinheits-Signalen von einer Authentifizierungseinheit (300) und zum Liefern der Authentifizierungseinheits-Signale als Steuersignale an die elektronische Schaltung (150) über die interne Steuerungs-Schnittstelle (140) ausgebildet ist, wobei die Authentifizierungseinheit (300) mit Mitteln zum Authentifizieren von autorisiertem Personal vor dem Übertragen der Steuersignale an die elektronische Schaltung (150) versehen ist;
wobei die Authentifizierungseinheits-Signale und die Steuersignale medizinische Auswahlinformationen der Geräte-Schnittstelle umfassen; und
wobei die medizinischen Auswahlinformationen der Geräte-Schnittstelle für eine oder mehrere der mehreren medizinischen Geräte-Schnittstellen (110, 120) einen verzögerten Aktivierungsbefehl umfassen;
wobei der verzögerte Aktivierungsbefehl erlaubt, dass ein jeweiliges der mehreren medizinischen Geräte (210, 220) mit einer medizinischen Geräte-Schnittstelle (110, 120) verbunden wird, ohne dass in einem vorbestimmten Zeitintervall irgendwelche Warnungen übertragen werden.

2. Verbindungseinheit (100) nach Anspruch 1, wobei die elektronische Schaltung (150) als einzelnes nicht-programmierbares elektronisches Gerät ausgeführt ist.

3. Verbindungseinheit (100) nach Anspruch 1 oder Anspruch 2, weiterhin umfassend eine Anzeige, die ausgebildet ist, einen Warnzustand zu signalisieren, wobei die elektronische Schaltung (150) weiterhin betriebsmäßig mit der Anzeige verbunden ist, und wobei die elektronische Schaltung (150) weiterhin ausgebildet ist, die Anzeige derart zu steuern, dass bei Empfang von Gerätewarnsignalen und Verlusten der Konnektivität der Warnzustand signalisiert wird.

4. Verbindungseinheit (100) nach Anspruch 3, wobei die Anzeige ausgebildet ist, die Warnung durch optische oder akustische Mittel zu signalisieren.

5. Verbindungseinheit (100) nach einem der vorstehenden Ansprüche, wobei die elektronische Schaltung (150) weiterhin ausgebildet ist, einen Verlust der Konnektivität an der Netz-Schnittstelle (130) zu überwachen und einen lokalen Warnzustand zu aktivieren, wenn der Verlust der Konnektivität an der Netz-Schnittstelle (130) erfasst wird.

6. Verbindungseinheit (100) nach Anspruch 5, weiterhin umfassend eine Raumlampen-Schnittstelle, wobei die elektronische Schaltung (150) weiterhin ausgebildet ist, ein Warnlichtsignal an die Raumlampen-Schnittstelle zu senden, wenn der lokale Warnzustand aktiviert wird.

7. Verbindungseinheit (100) nach einem der vorstehenden Ansprüche, wobei die medizinischen Auswahlinformationen der Geräte-Schnittstelle für eine oder mehrere der mehreren medizinischen Geräte-Schnittstellen einen aus einer Gruppe eines Aktivierungsbefehls und eines Deaktivierungsbefehls umfassen.

8. System, umfassend eine Verbindungseinheit (100) nach einem der vorstehenden Ansprüche und wenigstens ein medizinisches Gerät (210, 220), das mit einer der medizinischen Geräte-Schnittstellen (110, 120) verbunden ist.

9. System nach Anspruch 8, weiterhin umfassend eine Authentifizierungseinheit (300), die betriebsmäßig mit der Authentifizierungseinheits-Schnittstelle verbunden ist.

## Revendications

1. Unité de connexion (100) pour connecter une pluralité de dispositifs médicaux à un réseau d'appel infirmier, l'unité de connexion comprenant :
- une pluralité d'interfaces de dispositif médical (110, 120), conçues pour recevoir des câbles respectifs se connectant à des dispositifs médicaux respectifs de ladite pluralité de dispositifs médicaux (210, 220) ;
- une interface de réseau (130), conçue pour se connecter audit réseau d'appel infirmier ;
- une interface de commande interne (140) ;
- des circuits électroniques (150), connectés de manière opérationnelle à ladite pluralité d'interfaces de dispositif médical (110, 120), à ladite interface de réseau (130) et à ladite interface de commande interne (140), lesdits circuits électroniques (150) étant configurés pour :
• surveiller la réception de signaux d'alarme de dispositif et les pertes de connectivité au niveau de ladite pluralité d'interfaces de dispositif médical (110, 120) ;
• relayer les signaux d'alarme de dispositif reçus et les pertes de connectivité détectées vers ledit réseau d'appel infirmier par l'intermédiaire de ladite interface de réseau (130) ; et
• activer et désactiver de manière sélective ladite surveillance et ledit relais lors de la réception de signaux de commande prédéterminés sur ladite interface de commande interne ; et
- une interface d'unité d'authentification externe configurée pour recevoir des signaux d'unité d'authentification en provenance d'une unité d'authentification (300) et pour fournir lesdits signaux d'unité d'authentification en tant que signaux de commande auxdits circuits électroniques (150) par l'intermédiaire de ladite interface de commande interne (140), de sorte que ladite unité d'authentification est munie de moyens pour authentifier du personnel autorisé, avant de transmettre lesdits signaux de commande auxdits circuits électroniques (150) ;
dans laquelle lesdits signaux d'unité d'authentification et lesdits signaux de contrôle comprennent des informations de sélection d'interface de dispositif médical ; et
dans laquelle lesdites informations de sélection d'interface de dispositif médical comprennent, pour une ou plusieurs desdites interfaces de dispositif médical (110, 120), un ordre d'activation différée ;
ledit ordre d'activation différée permettant à un dispositif médical respectif de ladite pluralité de dispositifs médicaux (210, 220) d'être connecté à une interface de dispositif médical (110, 120) sans relayer aucune alarme pendant un intervalle de temps prédéterminé.

2. Unité de connexion (100) selon la revendication 1, dans laquelle lesdits circuits électroniques (150) sont mis en œuvre sous la forme d'un unique dispositif électronique non programmable.

3. Unité de connexion (100) selon la revendication 1 ou la revendication 2, comprenant en outre un indicateur conçu pour signaler une condition d'alarme, dans laquelle lesdits circuits électroniques (150) sont en outre connectés de manière opérationnelle audit indicateur, et dans laquelle lesdits circuits électroniques (150) sont en outre configurés pour commander ledit indicateur pour signaler ladite condition d'alarme lors de ladite réception de signaux d'alarme de dispositif et de pertes de connectivité.

4. Unité de connexion (100) selon la revendication 3, dans laquelle ledit indicateur est conçu pour signaler ladite alarme par des moyens visuels et/ou auditifs.

5. Unité de connexion (100) selon l'une quelconque des revendications précédentes, dans laquelle lesdits circuits électroniques (150) sont en outre configurés pour surveiller une perte de connectivité au niveau de ladite interface de réseau (130), et pour activer un état d'alarme locale lorsque ladite perte de connectivité au niveau de ladite interface de réseau (130) est détectée.

6. Unité de connexion (100) selon la revendication 5, comprenant en outre une interface de voyant de chambre, dans laquelle lesdits circuits électroniques (150) sont en outre configurés pour envoyer un signal lumineux d'alarme à ladite interface de voyant de chambre lorsque ledit état d'alarme locale est activé.

7. Unité de connexion (100) selon l'une quelconque des revendications précédentes, dans laquelle lesdites informations de sélection d'interface de dispositif médical comprennent, pour une ou plusieurs de ladite pluralité d'interfaces de dispositif médical, l'un d'un groupe d'un ordre d'activation et d'un ordre de désactivation.

8. Système comprenant une unité de connexion (100) selon l'une quelconque des revendications précédentes et au moins un dispositif médical (210, 220) connecté à l'une desdites interfaces de dispositif médical (110, 120).

9. Système selon la revendication 8, comprenant en outre une unité d'authentification (300) connectée de manière opérationnelle à ladite interface d'unité d'authentification.
